# EUROPEAN PATENT SPECIFICATION

(11) **EP 4 356 131 B1**
(45) Date of publication and mention of the grant of the patent: **30.07.2025**
(21) Application number: 22733946.2
(22) Date of filing: 15.06.2022
(51) Int. Cl.: G01N 33/543

(54) **METHOD FOR DIGITAL IMMUNOSENSING ON SINGLE MOLECULES USING LABEL IMMOBILIZATION AND AMPLIFICATION STRATEGY**
VERFAHREN ZUM DIGITALEN IMMUNOSENSING AN EINZELNEN MOLEKÜLEN UNTER VERWENDUNG VON LABELIMMOBILISIERUNG UND AMPLIFIKATIONSSTRATEGIE
PROCÉDÉ D'IMMUNODÉTECTION NUMÉRIQUE SUR DES MOLÉCULES INDIVIDUELLES UTILISANT L'IMMOBILISATION D'ÉTIQUETTE ET UNE STRATÉGIE D'AMPLIFICATION

(30) Priority: 17.06.2021 EP 21180002
(43) Date of publication of application: 24.04.2024
(73) Proprietor: F. Hoffmann-La Roche AG, 4070 Basel (CH); Roche Diagnostics GmbH, 68305 Mannheim (DE)
(72) Inventor: STENGELE, Nikolaus-Peter, 82377 Penzberg (DE)
(74) Representative: Altmann Stößel Dick Patentanwälte PartG mbB
(86) International application number: PCT/EP2022/066333
(87) International publication number: WO 2022/263522

(56) References cited:
- WO-A2-2008/086054
- US-A1- 2019 376 963
- US-B2- 7 635 571
- US-B2- 9 261 500

## Description

The present invention relates to diagnostic test and technology. In particular, the present invention relates to a method for determining an analyte suspected to be present in a sample comprising (a) contacting said sample with (i) a first binding agent which is capable of specifically binding to the analyte and which is immobilized on a solid support and (ii) a second binding agent which is capable of specifically binding to the analyte and which is capable of reversibly binding at least one detectable label, wherein said first and/or second binding agent is linked to an linking agent which is capable of covalently binding the at least one reversibly bound detectable label to the solid support when the first and second binding agents are in physical proximity, for a time and under conditions which allow specific binding of the analyte to the first and the second binding agent such that an complex of first binding agent, analyte and second binding agent is formed, and which allow for covalently binding the at least one detectable label to the solid support, and (b) removing the complex of first binding agent, analyte and second binding agent from the solid support such that the solid support having covalently bound the at least one detectable label remains, and (c) detecting the at least one covalently bound detectable label on the solid support whereby the analyte is determined. The present invention also relates to a device for determining an analyte suspected to be present in a sample, to the use of the device of the invention for determining an analyte suspected to be present in a sample in said sample, and to a kit for determining an analyte suspected to be present in a sample.

Immunoassays are widely used for various diagnostic purposes. Several setups have been developed for immunoassays. One of the most popular immunoassays is the enzyme-linked sorbent immunoassay (ELISA).

In ELISA, a liquid sample containing an anylte of interest or suspected to contain an anlyte of interest is applied onto a stationary solid phase with special binding properties due to the presence of antibodies or antibody-like molecules such as aptamers. After sample application, multiple reagents are sequentially added, incubated, and washed away in order to carry out and stop an anytical detection reaction. After carrying out all these steps, physical or chemical properties in the setup, usually in the liquid phase, are changed that can be detected. Typically, optical change such as color development by the product of an enzymatic reaction will happen in the final liquid phase. These changes correlate to the presence or abundance of the analyte of interest present in the investigated sample. The typically quantitative read-out is usually based on detection of intensity of transmitted light by spectrophotometry, which involves quantitation of transmission of some specific wavelength of light through the liquid. The sensitivity of detection depends on amplification of the signal during the analytic reactions. Since enzyme reactions are very well known amplification processes, the signal is generated by enzymes which are linked to the detection reagents in fixed proportions to allow accurate quantification.

For an ELISA setup, the analyte binding agent, e.g., an antibody, is immobilized on a soild phase such as a solid support structure. Usually, the antibody is coated and dried onto the transparent bottom and sometimes also side wall of a well in an analytic multi-well plate or in an analytic vial. However, nanoparticles or other beads can also be used as solid phases for ELISAs.

For research and diagnostic applications, ELISAs are often used in the format of so-called sandwich ELISAs. In a sandwich format, an immobilized capture antibody is used to capture, i.e. specifically bind to, an analyte present in a sample applied to said immobilized antibody. After the capture antibody has specifically bound to the analyte, the sample material is washed away. In a subsequent step the analyte bound to the immobilized antibody is incubated with a detection antibody that specifically binds to the analyte or the complex of analyte and capture antibody. The detection antibody typically contains a detectable linker or an adaptor molecule which allows for attracting such detectable labels from a solution.

However, in light of the fragile immune complexes which are required for signal generation and the various components used for such an ELISA in the sandwich format, there are many possibilities for undesired binding of detection antibodies and, thus, for the generation of false positive signals. Therefore, sandwich ELISAs used for research often need validation because of the risk of false positive results. Moreover, there are limitations with respect to sensitivity and specificity due to the various drawbacks of such a fragile multi-component assay format.

Document US2019/376963 refers to a method for detecting an analyte in a sample, the method applying first and second binding agents binding to the analyte on a first solid support, one of the binding agents comprising a detachable detectable label. The detachable detectable label is separated from the complex and is detected on a second solid support using a third binding agent specifically binding the detectable label.

The technical problem underlying the present invention may be seen as the provision of means and methods for complying with the aforementioned needs. The technical problem is solved by the embodiments characterized in the claims and herein below.

Thus, the present invention relates to a method for determining an analyte suspected to be present in a sample comprising:
(a) contacting said sample with
   (i) a first binding agent which is capable of specifically binding to the analyte and which is immobilized on a solid support; and
   (ii) a second binding agent which is capable of specifically binding to the analyte and which is capable of reversibly binding at least one detectable label;

   wherein said first and/or second binding agent is linked to an linking agent which is capable of covalently binding the at least one reversibly bound detectable label to the solid support when the first and second binding agents are in physical proximity,
   for a time and under conditions which allow:
      - specific binding of the analyte to the first and the second binding agent such that an complex of first binding agent, analyte and second binding agent is formed; and
      - which allow for covalently binding the at least one detectable label to the solid support; and
(b) removing the complex of first binding agent, analyte and second binding agent from the solid support such that the solid support having covalently bound the at least one detectable label remains; and
(c) detecting the at least one covalently bound detectable label on the solid support whereby the analyte is determined.

It is to be understood that in the specification and in the claims, "a" or "an" can mean one or more, depending upon the context in which it is used. Thus, for example, reference to "an" item can mean that at least one item can be utilized.

As used in the following, the terms "have", "comprise" or "include" are meant to have a non-limiting meaning or a limiting meaning. Thus, having a limiting meaning these terms may refer to a situation in which, besides the feature introduced by these terms, no other features are present in an embodiment described, i.e. the terms have a limiting meaning in the sense of "consisting of" or "essentially consisting of". Having a non-limiting meaning, the terms refer to a situation where besides the feature introduced by these terms, one or more other features are present in an embodiment described.

Further, as used in the following, the terms "preferably", "more preferably", "most preferably", "particularly", "more particularly", "typically", and "more typically" or similar terms are used in conjunction with additional or alternative features, without restricting alternative possibilities.

Further, it will be understood that the term "at least one" as used herein means that one or more of the items referred to following the term may be used in accordance with the invention. For example, if the term indicates that at least one item shall be used this may be understood as one item or more than one item, i.e. two, three, four, five or any other number. Depending on the item the term refers to the skilled person understands as to what upper limit the term may refer, if any.

The method according to the present invention may consist of the aforementioned steps (a) and (c) or may comprise further steps, such as pretreating or isolating the sample prior to step (a) and/or after step (c) further one or more steps of evaluating the determined analyte, e.g., by comparing it to references in order to provide a diagnostic, prognostic, environmental-relevant, agricultural-relevant or analytically-relevant conclusion depending on the aim of the determination of the analyte.

The term "determining" as used herein encompasses any kind of qualitative or quantitative determinations of the analyte. A qualitative determination aims at determining the presence or the absence of the analyte in the sample whereas quantitative determination aims at determining the amount of the analyte. The quantitative determination, i.e. the determination of the amount, includes determining the absolute amount (e.g. the total amount by weight or number of molecules present in the sample) or a relative amount (e.g., an amount relative to the sample volume (concentration) or a classification such as a score (e.g., "high amount", "low amount" and the like). Typically, determining an analyte comprises determining the presence or absence of said analyte or the amount of said analyte. More typically, the amount is determined quantitatively by using a "digital" detection as specified elsewhere herein.

The term "analyte" as referred to herein relates to any type of molecule or agent which is suitable for being determined by the method of the invention. It will be understood that such a molecule or agent may have a size and/or structure that allows binding of the first and second binding agents referred to herein. Moreover, there may be upper size limitations as well since the first and second binding agents as well as the linking agent must be capable of exert their functions as described elsewhere herein in detail. Typically, the analyte referred to herein is a biological molecule such as a protein, a peptide, a nucleic acid, e.g., a DNA or RNA or a small molecule such as a lipid or metabolite, a polyketide including, e.g., flavonoids and isoflavonoids, an isoprenoid including, e.g., terpenes, sterols, steroids, carotenoids, xanthophylls, a carbohydrate, a phenylpropanoide, an alcaloide, a benzenoide, an indole, a porphyrine, a hormone, a vitamin, a cofactor, a lignin, a glucosinolate, a purine, a pyrimidine, a nucleoside, a nucleotide, an alcohol, an alkane, an alkene, an alkine, an aromatic compound, a ketone, an aldehyde, a carboxylic acid, a ester, an amine, an imine, an amide, a cyanide, an amino acid, a thiol, a thioester, a phosphate ester, a sulfate ester, a thioether, a sulfoxide or an ether. The small molecule may be, e.g., a toxin. However, the method of the invention can also be used for determining viruses or even bacterial cells as analytes. Analytes to be determined by the present invention may also be molecules which are present in environmental samples and which may be useful as indicators for, e.g., environmental pollution, agriculture or other environmental conditions. Typically, said analyte is a protein, peptide, virus, bacterial cell or small molecule, preferably, small molecule toxin.

The term "sample" as used herein relates to any part or aliquot of a composition comprising or suspected to comprise the analyte to be determined. Such a sample may be a biological sample, typically, isolated from an organism, such as a body fluid or biopsy sample, or may be a composition comprising organism such as a cultured cells. Typically, said biological sample is investigated by the method of the invention for medical purposes, such as diagnosing or predicting a disease or medical condition. Moreover, the sample may be an environmental sample or an artificial sample. An environmental sample may be derived from any non-biological, natural source, e.g., environmentally occurring solutions such as water or from compositions such as soil. An artificial sample may be a sample obtained from an artificial source, e.g., a product composition which may be manufactured or an intermediate composition which may occur during a manufacturing process for a product. Such artificial samples may be investigated, e.g., for quality control purposes or for determining the amount of specific ingredients. Typically, said sample in accordance with the method of the present invention is a biological sample, preferably, a body fluid or biopsy sample.

The term "first binding agent" relates to a molecule which is capable of specifically binding to the analyte, i.e. a molecule which does not bind to and, thus, does not cross-react with other molecules than the analyte suspected to be present in the sample. Specific binding, in principle, can be tested by techniques well known in the art including screening assays for identifying agents specifically binding an analyte from libraries comprising different candidate agents.

The first binding agent in accordance with present invention shall be immobilized on a solid support. Immobilization of said first binding agent can be a permanent immobilization or a reversible immobilization. Permanent immobilization may be achieved by techniques well known in the art. However, it is envisaged that the immobilized first detection agent can be released from the solid support. In the case of antibodies or other proteins or peptides used as first detection agents, this may be achieved by specific proteolytic cleavage. I the case of nucleic acid based aptamers, removing a first binding agent immobilized on a solid support from said solid support may be achieved by nuclease digestion. Typically, the immobilization is a reversible immobilization, e.g., an immobilization achieved by nucleic acid hybridization such as L-DNA capture.

Molecules which may be used, preferably, as first binding agents being capable of specifically binding a desired analyte may be antibodies. Antibodies as binding agents as meant in accordance with the present invention encompass to all types of antibodies which, preferably, specifically binds to the analyte. Preferably, an antibody of the present invention may be a monoclonal antibody, a polyclonal antibody, a single chain antibody, a chimeric antibody or any fragment of such antibodies being still capable of specifically binding to the analyte. Such fragments comprised by the term antibody as used herein encompass a bispecific antibody, a synthetic antibody, an Fab, F(ab)₂ Fv or scFv fragment or a chemically modified derivative of any of these antibody fragments. Antibodies or fragments thereof, in general, which specifically bind to a desired analyte can be obtained by using methods which are described, e.g., in Harlow and Lane "Antibodies, A Laboratory Manual", CSH Press, Cold Spring Harbor, 1988. Monoclonal antibodies can be prepared by the techniques which comprise the fusion of mouse myeloma cells to spleen cells derived from immunized mammals and, preferably, immunized mice (Köhler 1975, Nature 256, 495, and Galfré 1981, Meth. Enzymol. 73,3). The skilled person is well aware of how specific binding can be tested by techniques well known in the art, such as immunological or biochemical techniques including immunoassays, cell sorting or Western blotting or plasmon surface resonance measurements.

Further, molecules which may be used, preferably, as first binding agents being capable of specifically binding a desired analyte may be aptamers. An aptamer as a binding agent in accordance with the present invention may be an oligonucleic acid or peptide molecule that binds to a specific target analyte (Ellington 1990, Nature 346 (6287): 818-22). Bock 1992, Nature 355 (6360): 564-6). Oligonucleic acid aptamers are engineered through repeated rounds of selection or the so-called systematic evolution of ligands by exponential enrichment (SELEX technology). Peptide aptamers usually comprise of a variable peptide loop attached at both ends to a protein scaffold. This double structural constraint shall increase the binding affinity of the peptide aptamer into the nanomolar range. Said variable peptide loop length is, preferably, composed of ten to twenty amino acids, and the scaffold may be any protein having improved solubility and compacity properties, such as thioredoxin-A. Peptide aptamer selection can be made using different systems including, e.g., the yeast two-hybrid system (see e.g., Hoppe-Seyler 2000, J Mol Med. 78 (8): 426-30). Also encompassed according to the present invention are any fragments of said aptamers that are still capable of specifically binding to the analyte. Said fragments can be used in isolated form or may be part of fusion molecules, i.e. molecules comprising said aptamer fragment as well as other parts such as linker moieties or adapter molecules. The skilled person is well aware of how specific binding can be tested by techniques well known in the art, such as plasmon surface resonance measurements.

Molecules which may be used, preferably, as first binding agents being capable of specifically binding a desired analyte may also be receptor molecules. A receptor molecule as a binding agent as referred to in accordance with the present invention is, typically, a protein that specifically binds to a ligand and which become activated upon ligand binding to exert its biological function. Such a receptor molecule or a fragment thereof being still capable of specifically binding to the ligand may also be used as a binding agent in accordance with the present invention for the ligand as analyte or a molecule which is derived from the ligand but still capable of being bound by the receptor molecule or fragment thereof such as antagonistically or agonistically acting variants of a ligand. Preferably, the receptor molecule envisaged as a binding agent in accordance with the present invention may be a transmembrane type receptor protein, such as a G-protein coupled receptor, e.g., a metabolic receptor, an enzyme linked receptor, such as a receptor tyrosine kinase, e.g., a growth factor receptor, an immunological receptor, such as a virus receptor, cell surface antigen, or a T cell receptor, e.g., CD4, CD3 or CD8, MHC proteins, a cell adhesion molecule, such as an integrin, cadherin, selectin or syndecan, a neuronal receptor, or a pathogen receptor such as Toll-like receptors. However the receptor molecule may be a nuclear receptor protein as well such as a nuclear hormone receptor, e.g., the glucocorticoid receptor, retinoic acid receptor or thyroid hormone receptor. The skilled person is well aware of receptor molecules or fragments thereof which specifically bind to an analyte to be determined or fragments thereof. Moreover, specific binding can be tested by techniques well known in the art, such as plasmon surface resonance measurements.

Yet, molecules which may be used, preferably, as first binding agents being capable of specifically binding a desired analyte may also be ligand molecules. A ligand molecule as a binding agent as referred to in accordance with the present invention is, typically, a protein or peptide that specifically binds to a receptor molecule and which activates upon receptor binding the said receptor molecule. Such a ligand molecule or a fragment thereof being still capable of specifically binding to the receptor molecule may also be used as a binding agent in accordance with the present invention for the receptor as analyte or a molecule which is derived from the receptor molecule but still capable of being bound by the ligand molecule or fragment thereof such as soluble variants of a receptor. Moreover, a ligand may also be any antigen which may be used to determine a certain antibody in a sample of an organism. Preferably, the ligand molecule envisaged as a binding agent in accordance with the present invention may be a peptide hormone, a neurotransmitter, a growth factor, such as angiopoietin, a BMP, a neutrotrophic factor, EGF, an epiphrin, EPO, a FGF, a GDNF, a GDF, insulin or an insulin-like growth factor, a TGF, a neutrophin, a VEGF, a cytokine, such as an interleukin, interferon, lymphokine, monokine, colony stimulating factor or chemokine, a extracellular matrix protein such as fibronectin, vitronectin, collagen, ankyrin or laminin, or the like. The skilled person is well aware of ligand molecules or fragments thereof which specifically bind to an analyte to be determined or fragments thereof. Moreover, specific binding can be tested by techniques well known in the art, such as plasmon surface resonance measurements.

Further preferably, the first binding agent may be a Designed Ankyrin Repeat Protein (DARPin). DARPins are genetically engineered antibody mimetic proteins which can be designed to achieve highly specific and high-affinity target protein binding. They are derived from natural ankyrin repeat proteins. Typically, DARPins comprise at least three repeat modules, of which the most N- and the most C-terminal modules (also referred to as "caps") protect the hydrophobic core of the protein (Binz 2003, Journal of Molecular Biology. 332 (2): 489-503).

Typically, said first binding agent is selected from the group consisting of: an antibody or fragment thereof, an aptamer, a receptor molecule or fragment thereof, and a ligand molecule or fragment thereof. More typically, it is an antibody or fragment thereof or an aptamer.

The term "solid support" as used herein relates to a solid composition of matter which can serve as a basis for immobilizing molecules and, in particular, the first binding agent and the detectable label. A solid support may comprise inorganic or organic compounds or both. Typically, suitable inorganic compounds for a solid support may be selected from the group consisting of: silica, porous glass, aluminosilicates, borosilicates, metal oxides (e.g. aluminum oxide, iron oxide, nickel oxide) and clay containing one or more of these. The solid support may also comprise conductive compounds such as a metal or graphite. Preferably, such a solid support may be an electrode, a semiconductor, a plasmonic resonator or any kind of electrical circuit arrangement. Alternatively, the solid support may comprise an organic compound such as a cross-linked polymer. Non-limiting examples of a suitable cross-linked polymer may be selected from the group consisting of: polyamide, polyether, polystyrene and mixtures thereof. The skilled artisan is well aware of how to select a suitable solid support based on the kind of sample to investigated, the method envisaged for detection of the analyte and the kind of first molecule to be used for detecting the analyte.

Typically, said solid support comprises on its surface a linker to which the label can be covalently bound by the linking agent. Typically, a liker to be used on the surface of the solid support may be immobilized peptides which may serve as K-tags for transglutaminases.

Yet, said solid support envisaged according to the present invention is, typically, an electrode or planar waveguide. Also typically, said solid support is a bead, well or predetermined subdivided detection area, preferably, of a plasmonic resonator. More typically, each bead, well or predetermined subdivided detection area has immobilized a predefined amount of first binding agent, preferably, at most 3, at most 2 or, most preferably, 1.

When determining an analyte comprises determining the amount of said analyte, typically, a bead, well or predetermined subdivided detection area, preferably, of a plasmonic resonator may be used, more preferably, having immobilized a predefined amount of first binding agent. In light of the predefined ratio of first binding agents to bead, well or predetermined subdivided detection area, it will be understood that a signal generated by one or more detectable labels on a bead or in a well or predetermined subdivided detection area correlates to the originally formed number of complexes of first binding agent, analyte and second binding agent. Thus, preferably, the analyte amount is determined by counting the beads, wells or predetermined subdivided detection areas which comprise at least one covalently bound detectable label. Subsequently, the number of originally formed complexes and thereby the number of analytes can be calculated based on the existing relationships.

The term "second binding agent" which is capable of specifically binding to the analyte relates to a molecule which is capable of specifically binding to the analyte the anaylte when bound to the first binding agent or the first binding agent when bound to the analyte, i.e. a molecule which does not bind to and, thus, does not cross-react with other molecules than the analyte or complex of analyte and first binding agent suspected to be present in the sample. Typically, said second binding agent is selected from the group consisting of: an antibody or fragment thereof, an aptamer, a receptor molecule or fragment thereof, and a ligand molecule or fragment thereof. More typically, it is an antibody or fragment thereof or an aptamer. Typically, the second binding agent is from the same of the aforementioned molecule class as the first binding agent. The definitions for an antibody or fragment thereof, an aptamer, a receptor molecule or fragment thereof, and a ligand molecule or fragment thereof made in accordance with the first binding agent apply mutatis mutandis for the second binding agent.

The term "detectable label" as used herein relates to molecules which exhibit physical or chemical properties which can be detected in the settings of the method of the present invention. Preferably, said properties are optically detectable properties such as emission of radiation, chemiluminescence, fluorescence or FRET, electromagnetic properties such as interference with electrical or magnetic fields, detectable radioactivity or chemical properties such as capability of performing or catalyzing a certain chemical reaction. The detectable label shall be reversibly bound to the second binding agent. To this end, the detectable label may be a molecule which is capable of reversibly binding to the second binding agent or it may be a molecule or moiety which is already part of the said second binding agent. Typical detectable labels include gold particles, latex beads, acridan ester, luminol, ruthenium, enzymatically active labels, radioactive labels, magnetic labels, or fluorescent labels. Enzymatically active labels include horseradish peroxidase, alkaline phosphatase, beta-Galactosidase or Luciferase. Suitable substrates for detection of such enzymatically detectable labels include di-amino-benzidine (DAB), 3,3'-5,5'-tetramethylbenzidine, NBT-BCIP (4-nitro blue tetrazolium chloride and 5-bromo-4-chloro-3-indolyl-phosphate. A suitable enzyme-substrate combination may result in a colored reaction product, fluorescence or chemiluminescence which can be measured according to methods known in the art, e.g., by using a light-sensitive film or a suitable camera system. Typical fluorescent labels include fluorescent proteins, such as GFP, RFP, YFP, BFP or variants thereof, Cy3, Cy5, Texas Red, Fluorescein, and the Alexa dyes, e.g., Alexa 568. Also the use of quantum dots as fluorescent labels is contemplated. Typical radioactive labels include 35S, 125I, 32P, 33P and the like. A radioactive label can be detected by any method known and appropriate, e.g. a light-sensitive film or a phosphor imager. Typical magnetic labels comprise iron-platinum nanoparticles, iron nanoparticles, nickel nanoparticles or cobalt nanoparticles. Typically, said detectable label is selected from the group consisting of: fluorescent labels, chemiluminescent label, radioactive labels, magnetic labels, and electrochemical labels. Typically, said detectable label comprises a linker which can be covalently linked by the linking agent to the solid support.

The term "linking agent" as referred to herein relates to an agent which is capable of covalently binding the detectable label to the solid support. It will be understood that the selection of the linking agent to be used in the method of the present invention is depending on the chemical nature of the detectable label and the solid support. The linking agent may be linked to the first and/or second binding agent directly or via a linker molecule. The linking agent may be linked covalently or it may be linked reversibly. Typically, a linking agent may be bound by a linker only after a complex of the first binding agent, analyte and second binding agent has been formed. In such a case, a split aptamer may be used wherein one part of the aptamer is bound to the first binding agent and a second part of the aptamer is bound to the second binding agent. Each of the aptamere parts alone is not capable of binding to the liniking agent. However, once the complex of first binding agent, analyte and second binding agent is formed, the aptamer shall specifically bind to the linking agent. Such split aptamers are known in the art (Walter 2017, Nano Lett 17, 2467-2472). Alternatively, if a cross-linking enzyme is used as a linking agent, the first and the second binding agents may be linked to different parts of the said enzyme which require the presence of each other in order to become functionally active. Typically, said linking agent may also be a covalently cross-linking enzyme, preferably, a transglutaminase or sortase or said linking agent comprises covalently cross-linking chemical moieties. The skilled artisan is well aware of how to choose a linking agent taking into account the chemical nature of the detectable label and/or the solid support.

Preferably, a transglutaminase may be used as linking agent. Transglutaminases are crosslinking enzymes that catalyze the formation of isopeptide bonds between γ-carboxamide groups of the side chains in glutamine residues and the ε-amino groups of side chains of lysine residues or the primary amine residue of a peptide or protein whereby ammonia is released (E.C. number EC 2.3.2.13). Typically, a transglutaminase envisaged in accordance with the present invention may transfer a Q-Tag on a detectable label to a K-tag peptide which is immobilized as a linker on the solid support. Transglutaminases are found in various organisms including bacteria, mammals and humans. In humans, the following transglutaminases have, inter alia, been reported: Factor XIII, keratinocyte transglutaminase, tissue-type transglutaminase, epidermal transglutaminase, prostate transglutaminase, TGM X, TGM Y, TGM Z, protein 4.2.

Preferably, a sortase may also be used as a linking agent. Sortases are bacterial enzymes that act as transpeptidases (E.C. number 3.4.22.70). In particular, they cleave a substrate protein or peptide in a conserved cleavage side and catalyze the formation of an amide bond between the carboxy terminal end resulting from cleavage and a free amino group of another protein or peptide, typically, a peptidoglycan. Preferably, the transpeptidase activity of a sortase may be used such that the cleavage side is added to the C-terminus of a first protein while an oligo-glycine motif is added to the N-terminus of a second protein to be ligated. Upon addition of sortase to the protein mixture, the two peptides are covalently linked through a native peptide bond. The term includes full-length sortase proteins, as well as fragments thereof having sortase activity. Typically, sortases suitable in accordance with the present invention include sortase A, sortase B, sortase C, and D type sortases.

Moreover, the linking agent, also preferably, may comprises covalently cross-linking chemical moieties. Typically, chemical moieties which are suitable as linking agent are present on the first and/or second binding agent and shall together chemically catalyze or enable covalent binding of the detectable label to the solid support. For example, the detectable label may be non-covalently bound to the complex by, e.g., an antibody or aptamer acting as a linking agent in that it brings upon formation of the complex of first binding agent, analyte, and second binding agent into functional proximity to the solid support. A chemical moiety present on the solid support and a chemical moiety present on the detectable label shall subsequently be capable of interacting with each other such that a covalent bound is formed between the detectable label and the solid support. Alternatively a first chemical moiety present on the second binding agent may be brought into functional proximity to the solid support which presents a second chemical moiety which is modified upon complex formation to become a third chemical moiety. Said third chemical moiety will subsequently be capable of covalently binding to a chemical moiety of the detectable label. Suitable chemical moieties for such approaches may be those which can be applied in click-chemistry reactions, such as cycloadditions, e.g., the Huisgen 1,3-dipolar cycloaddition, Thiol-ene reactions, Diels-Alder reactions, inverse electron demand Diels-Alder reactions, nucleophilic substitutions, carbonyl-chemistry-like formation of ureas or addition reactions to carbon-carbon double bonds like dihydroxylation or the alkynes in the thiol-yne reaction. Typically, such reactions are carried out as biorthogonal reactions, i.e. the recants of the reaction should be kinetically, thermodynamically, and metabolically stable before the reaction in the system and should not interfere with other reactions or components of the system. Both reactants shall interact specifically with each other, i.e. no cross-reactivity shall take place in the system. The conditions for the reactions of said reactants shall not interfere with the stability of other components or reactions in the system.

In step (a) according to the method of the preset invention, a sample which comprises the analyte to be determined or which is suspected to comprise the analyte to be determined is brought into contact with an arrangement comprising on a solid support a first binding agent which is capable of specifically binding to the analyte and which is immobilized on a solid support. Said arrangement may be provided as a sensor in accordance with the present invention as described elsewhere herein.

The term "contacting" as used herein relates to bringing the aforementioned components in physical proximity such that the first and/or second binding agent may bind to the analyte if present in the sample. It will be understood that binding of the first binding agent to the analyte and of the second binding agent to the analyte or the complex of first binding agent and analyte may require time and applying suitable conditions. The skilled person is well aware of what time is required for achieving binding and what conditions need to be applied. For example, the sample and the binding agents may be dissolved or mixed with buffers adjusting salt concentrations and/or pH value. It will be understood that suitable buffers and other auxiliary components which may be applied depend on the binding agents to be used and the chemical nature of the analyte to be determined.

In step (a) of the method of the present invention, the first binding agent and the analyte are also brought into contact to a second binding agent which is capable of specifically binding to the analyte and which is capable of reversibly binding at least one detectable label. The second binding agent may be applied after the sample has been brought into contact with the first binding agent immobilized on the solid support. Alternatively, the sample may be brought into contact with the first binding agent together with the second binding agent, i.e. both components may be applied to the solid support and the immobilized first binding agent simultaneously. Yet as an alternative, the second binding agent and the sample may be mixed and the resulting mixture will be subsequently applied to the solid support comprising the immobilized first binding agent.

Moreover, in step (a) of the method of the present invention, a detectable label shall be applied to the sample an the first and second binding agent. This can be done together when the sample is being contacted with the first and/or second binding agent as described before or after said components have been contacted with each other.

The first and/or the second binding agent applied in the method of the present invention shall be linked to a linking agent which is capable of covalently binding the at least one reversibly bound detectable label to the solid support when the first and second binding agents are in physical proximity. As described before, this is the case if the complex between the first binding agent, the analyte and the second binding agent is formed and the linking activity of the linking agent is present such that the detectable label can be covalently linked to the solid support. Accordingly, step (a) of the method further requires to be carried out for a time and under conditions which allow for, both, specific binding of the analyte to the first and the second binding agent such that an complex of first binding agent, analyte and second binding agent is formed and for covalently binding the at least one detectable label to the solid support.

The detectable label may be covalently linked to the solid support or to a liker molecule immobilized on the solid support. Dependent on the chemical nature of the detectable label, the skilled artisan is well aware of suitable linkers for a detectable label or means for directly linking a detectable label to the solid support.

As a result of the aforementioned activities which occur during step (a) of the method of the present invention, at least one detectable label will become covalently bound to the solid support. Using enzymes as linking agents, it will be understood that as a result of one complex formation caused by the presence of one analyte, more than one detectable label shall be immobilized by covalent binding on the solid support. Thus, an event of complex formation due to the presence of one analyte molecule in a sample may be amplified to cause various immobilizations of detectable label molecules on the solid support which allows for easy and accurate detection.

In step (b) of the method of the present invention, the complex of first binding agent, analyte and second binding agent is removed from the solid support such that the solid support having covalently bound the at least one detectable label remains.

The term "removing" as used herein relates to any measure which removes the entire complexes formed in step a or parts thereof from the solid support such that the linking agent is no longer capable of reversible binding to the detectable label and/or to immobilize it by covalent binding on the solid support. Typically, removing as referred to herein is achieved by washing the solid support with washing solution such as buffers which apply conditions that dissolve the complex or components thereof. Alternatively or in addition, temperature or other physical forces may be applied, e.g., shaking or centrifugation. However, it will be understood that the covalently bound detectable label remains bound on the solid support and is not affected by the measures used for removing the complex. Suitable measures for removing the complex, typically, depend on the nature of the immobilization of the first binding agent to the solid support. If the first binding agent is immobilized, e.g., by nucleic acid hybridization, applying temperature and/or salts that promote dissociation of the hybridized nucleic acid strands may be used for removing the complex. If the first binding agent is an immobilized antibody or fragment thereof, receptor protein or fragment thereof or ligand protein or fragment thereof, a proteolytic digest using a protease that cleaves-off the immobilized antibody or fragment thereof, receptor protein or fragment thereof or ligand protein or fragment thereof from the solid support may be used. Similarly, if a nucleic acid-based aptamer is used as a first binding agent, a nuclease digestion may be used to cleave off the aptamer from the solid support.

As a result of step (b), the solid support shall be free of intact complexes and only covalently bound detectable labels shall remain. Said remaining detectable labels represent former complex formation events. Moreover, detectable labels which may generate unspecific noise signals shall also be largely removed as well as second binding agents and other components that may interfere with proper signal detection elicited by covalently bound detectable labels.

In step (c ) of the method of the present invention, the at least one covalently bound detectable label on the solid support is detected whereby the analyte is determined.

The term "detecting" as used herein relates to identifying the presence, absence and/or amount of label molecules covalently bound to the solid support by measuring a physical-, chemical-, physicochemical- and/or biological property of said label molecules. It will be understood that the detectable label typically generates a signal that can be measured. The signal strength and/or duration is, typically, indicative for the amount of the detectable label molecules present on the solid support. Depending on the chemical nature of the detectable label, the signal may be an active signal such as emission of light or other radiation, or it may be a passive signal, i.e. a signal which is elicited as a response to an external stimulus such as applying an electromagnetic field, radiation or others. The skilled person is well aware of how measurement of such signals elicited by the detectable label molecules can be carried out and it will be understood that the detection method to be used in accordance with the method of the present invention depend on the detectable label used. Preferred measurement methods according the present invention also include chemiluminescence measurements, fluorescence measurements, FRET measurements, electrochemiluminescence measurements, mass measurements including mass spectrometry, magnetic or electrical filed-based measurements using, e.g., ChemFETs or other electrode arrangements, radioimmunoassay measurements, dissociation-enhanced lanthanide fluoro immunoassay (DELFIA) measurements, scintillation proximity assay measurements, quantum dot technology measuremets, turbidimetry measurements or nephelometry measurements.

The detectable label may also be detected indirectly, i.e. by using a further labeled molecule that is capable of binding specifically to the immobilized detectable label on the solid support. Such a further labeled molecule may be applied to the solid support after step (b) has been carried out and prior to step (c). It will be understood that upon specific binding of one or more of said further labeled molecule to the detectable label(s) on the solid support, said further labeled molecules may be detected in step (c) by measuring a physical-, chemical-, physicochemical- and/or biological property of said labeled molecules. Yet, a further molecule that is capable of specifically binding to the immobilized detectable label may also serve as an anchor for other labeled molecules. Suitable molecules which may serve as further labeled molecules or anchor molecules may also be antibodies, aptamers or other molecules which allow for specific binding of a target as descried elsewhere herein. Suitable labels for such further molecules are those described for the detectable label elsewhere herein.

In the method of the present invention, first and second binding agents may be used for determining different analytes. In such a case, it will be understood that once the complexes for such different analytes are formed, each complex must possess a different linking agent and provide for different linking activity for a different detectable label. For example, an analyte A shall be determined by using first and second detectable labels A linked to linking agent A which subsequently upon complex A formation covalently binds detectable label A to the solid support and an analyte B shall be subsequently or simultaneously determined by using first and second detectable labels B linked to linking agent B which subsequently upon complex B formation covalently binds detectable label B to the solid support. Typically, detectable labels A and B which are present on the solid support if analytes A and B were present in the sample to be investigated can be detected by using detectable a physical-, chemical-, physicochemical- and/or biological property which differs between said detectable labels.

In a particular embodiment of the method of the present invention, said solid support is a bead, well or predetermined subdivided detection area, preferably, of a plasmonic resonator. Preferably, each bead, well or predetermined subdivided detection area has immobilized on it a predefined amount of first binding agent. Said method is particularly useful for determining an analyte comprises determining the amount of said analyte. Typically, said amount is determined by counting the beads, wells or predetermined subdivided detection areas which comprise at least one covalently bound detectable label. According to this particular embodiment, the presence or absence or the amount of detectable label covalently bound to said bead, well or predetermined subdivided detection area is detected for each bead, well or predetermined subdivided area. Afterwards, the number of positive beads, wells or predetermined subdivided areas shall be determined. A positive bead, well or predetermined subdivided area shall be one which exhibits detectable label or which exhibits detectable label eliciting a measurable signal of a predetermined strength or duration, e.g., a measurable signal above a predetermined strength threshold or over a predetermined time window. Based on the positive beads, wells or predetermined subdivided areas and the predefined amount of first binding agent immobilized on each of said beads, wells or predetermined subdivided areas, the amount of analyte which caused the covalent binding of the detectable label to the beads, wells or predetermined subdivided areas can be determined, e.g., by calculations. For example, if each bead, well or predetermined subdivided area contains ideally one first binding agent, the presence of one molecule of analyte in a sample shall generate one complex which causes covalent binding of at least one detectable label to said well, bead or predetermined subdivided area. Thus, if the presence of detectable label(s) on a bead, well or predetermined subdivided area is determined, this presence reflects the presence of one analyte present in the sample. Thus, using a predefined amount of first binding agents on isolated items such as beads, wells or any other predetermined subdivided areas, allows for quantitative or semi-quantitative determination of analyte molecules present in a sample. This technique is also called "digital" detection.

Advantageously, it has been found in accordance with the present invention that using covalent binding of a detectable label to a solid support as a result of immune complex formation in a sandwich assay format may be used to improve sensitivity and to suppress undesired background noise. In accordance with the present invention, the covalent binding of detectable labels to a solid support only occurs if the immune complex comprising a first binding agent, e.g., antibody, an anlyte to be determined, a second binding agent, e.g., antibody is formed and the first and the second binding agents due to their participation in the complex acquire or activate a linking agent that enables covalent linkage of detectable labels to the solid support. Thereby, a single immune complex formation event resulting from the presence of a single analyte molecule in a sample to be investigated will cause covalent binding of several detectable labels to the solid support and, thus, enhance signals elicited by the detectable label which are representative for the presence of said analyte molecule. Noise causing components may be removed after the detectable label(s) have been covalently linked to the solid support efficiently. Depending on the nature of the solid support, it is possible to essentially singularize the complex formation events. E.g., if a bead is used as solid support having immobilized on its surface a single first binding agent initiating the complex formation in the presence of one analyte molecule, it will be understood that if said bead after carrying out complex formation and removal of noise components comprises one or more covalently bound detectable labels on its surface, said labeled bead shall represent the one analyte molecule present in the sample. In such a "digital" detection format, the number of labeled beads may be used to count the analyte molecules present in the sample.

Thanks to the present invention, determination of an anaylte in a sample with an ultimate sensitivity on the level of molecule counts is possible. Due to the covalent linkage of the detectable labels to the solid support, noise-causing components such s unbound detectable labels and/or first and second binding agents can be efficiently removed by harsh washing or other treatments.

The present invention also relates to a device for determining an analyte suspected to be present in a sample comprising
(a) a sensor element comprising:
   (i) a first binding agent which is capable of specifically binding to the analyte and which is immobilized on a solid support; and
   (ii) a second binding agent which is capable of specifically binding to the analyte and which is capable of reversibly binding at least one detectable label;
   wherein said first and/or second binding agent is linked to an linking agent which is capable of covalently binding the at least one reversibly bound detectable label to the solid support when the first and second binding agents are in physical proximity; and
(b) a detector for detecting the at least one covalently bound detectable label on the solid support.

The term "device" as used herein relates to a system comprising the aforementioned components operatively linked to each other as to allow the determination of the analyte according to the method of the invention.

The sensor element, typically, comprises a reaction zone having said first binding agent which is capable of specifically binding to the analyte and which is immobilized on a solid support which is to be contacted to the sample comprising the analyte or suspect to comprise the analyte. Moreover, in the reaction zone, it is possible to apply the second binding agent, the detectable label and conditions which conditions which (i) allow specific binding of the analyte to the first and the second binding agent such that an complex of first binding agent, analyte and second binding agent is formed and (ii) allow for covalently binding the at least one detectable label to the solid support.

Moreover, the reaction zone shall allow for applying conditions for removing from the solid support the complex of first binding agent, analyte and second binding agent from the solid support such that the solid support having covalently bound the at least one detectable label remains. As described elsewhere herein such conditions may include washing with washing solutions, such as suitable buffers or other solvents, and/or the application of temperature or other physical forces.

The reaction zone may either allow directly for sample application or it may be connected to a loading zone where the sample is applied. In the latter case, the sample can be actively or passively transported via the connection between the loading zone and the reaction zone to the reaction zone. Moreover, the reaction zone shall be also connected to a detector. The connection shall be such that the detector can detect the detectable labels covalently bound to the solid support. Suitable connections depend on the techniques used for measuring the presence or amount of detectable label(s). For example, for optical detection, transmission of light may be required between the detector and the reaction zone while for electrochemical determination a fluidal connection may be required, e.g., between the reaction zone and an electrode.

The detector shall be adapted to detect the presence or amount of the covalently bound detectable label on the solid support. Depending on the detectable label, different detection techniques may be applied.

Optically detectable labels may be determined by measuring light emission, fluorescence, FRET, polarization, refraction and the like. Typical optical detectors may be photomultipliers, phototubes, ionization detectors, active-pixel sensors, phototransistors, photodiodes, quantum dot photoconductors or photodiodes, photovoltaic cells, semiconductor detectors, thermal detectors, photochemical detectors and the like.

Electrochemically detectable labels may be determined by electrode arrangements such as thin layer electrodes suitable for voltametric and, typically, amperometric measurements. The detector, typically, contains at least two electrodes of which at least one electrode is a so-called working electrode. The electrodes can be composed of all conventional electrode materials such as metals, noble metals, alloys or graphite and are preferably composed of noble metals such as gold or palladium, or graphite. The various electrodes of the sensor can be composed of the same or different materials. More typically, the electrode are made of palladium.

It will be understood that the detected presence, absence or amount of the detectable label covalently bound to the solid support can be subsequently transmitted to an evaluation unit. Said evaluation unit may, preferably, comprise a data processing element, such as a computer, with an implemented algorithm for determining the presence absence or amount of an anylte in the sample based on the detected presence, absence or amount of detectable label(s).

Such an implemented algorithm may evaluate the measured signals elicited from the detectable label(s) for signal strength, signal duration and other predetermined parameters. Based on said evaluation, truly positive signals can be identified and validated and noise signals can be identified and disregarded for further evaluations. The skilled artisan is well aware of what algorithms may be used and how they can be implemented in the device of the present invention.

Moreover, an evaluation unit may also comprise an implemented algorithm for identifying individual beads, wells or predetermined subdivided detection areas and counting the beads, wells or predetermined subdivided detection areas which comprise at least one covalently bound detectable label and thereby determining the amount of the analyte.

The present invention, in general, relates to the use of the device of the invention for determining an analyte suspected to be present in a sample in said sample. More typically, the device is used for determining the analyte by carrying out the method of the invention described elsewhere herein in more detail.

The present invention, furthermore, relates to a kit for determining an analyte suspected to be present in a sample comprising:
(a) a first binding agent which is capable of specifically binding to the analyte and which is immobilized on a solid support;
(b) a second binding agent which is capable of specifically binding to the analyte and which is capable of reversibly binding at least one detectable label; wherein said first and/or second binding agent is linked to an linking agent which is capable of covalently binding the at least one reversibly bound detectable label to the solid support when the first and second binding agents are in physical proximity,
(c) a detectable label; and
(d) an agent for removing the complex of first binding agent, analyte and second binding agent from the solid support such that the solid support having covalently bound the at least one detectable label remains.

The term "kit" as used herein refers to a collection of the aforementioned components, typically, provided in separately or within a single container. The container also typically comprises instructions for carrying out the method of the present invention. These instructions may be in the form of a manual or may be provided by a computer program code which is capable of carrying out or supports the determination of fibrinogen referred to in the methods of the present invention when implemented on a computer or a data processing device. The computer program code may be provided on a data storage medium or device such as an optical storage medium (e.g., a Compact Disc) or directly on a computer or data processing device or may be provided in a download format such as a link to an accessible server or cloud. Moreover, the kit may, usually, comprise analyte solutions with standardized amounts for calibration or validation or other reference amounts. The kit according to the present invention may also comprise further components which are necessary for carrying out the method of the invention such as washing solutions, solvents, and/or reagents required for detection of the detectable label. Further, it may comprise the device of the invention either in parts or in its entirety.

### FIGURES

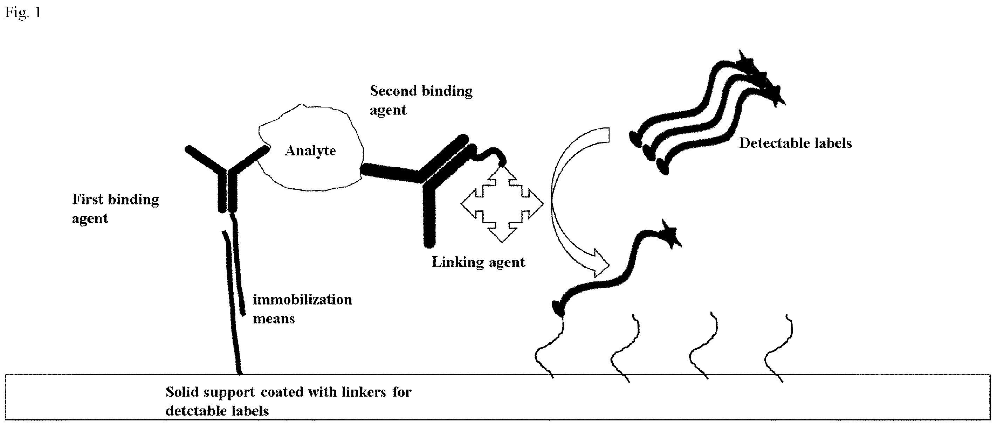

**Figure 1****:** A schematic setup of the sandwich assay implementing the method of the invention is shown. On a solid support, there are linkers for immobilizing the first binding agent, e.g., a first antibody, which is capable of specifically binding to an analyte of interest. This linker, e.g., a nucleic acid (LLNA), is capable of binding to a moiety or adapter on the first binding agent in order to immobilize the first binding agent on the solid support. The first binding agent, e.g., a first antibody, specifically binds to the analyte of interest. The analyte of interest when bound by the first binding agent will be specifically bound by the second binding agent, e.g., a second antibody. The second binding agent is linked to a linking agent, e.g., a transglutaminase. Once being part of the immune complex, the second binding agent brings into proximity to the solid support the linking agent. The linking agent, e.g., a transglutaminase covalently binds detectable labels to the surface of the solid support. This can be achieved by using Q-tags on the label molecules that are transferred and covalently bound to K-tags present as linkers on the solid support. Detectable labels that are covalently bound to the solid support will later be detected in the method of the invention after the immune complex has been removed, e.g., by washing steps.

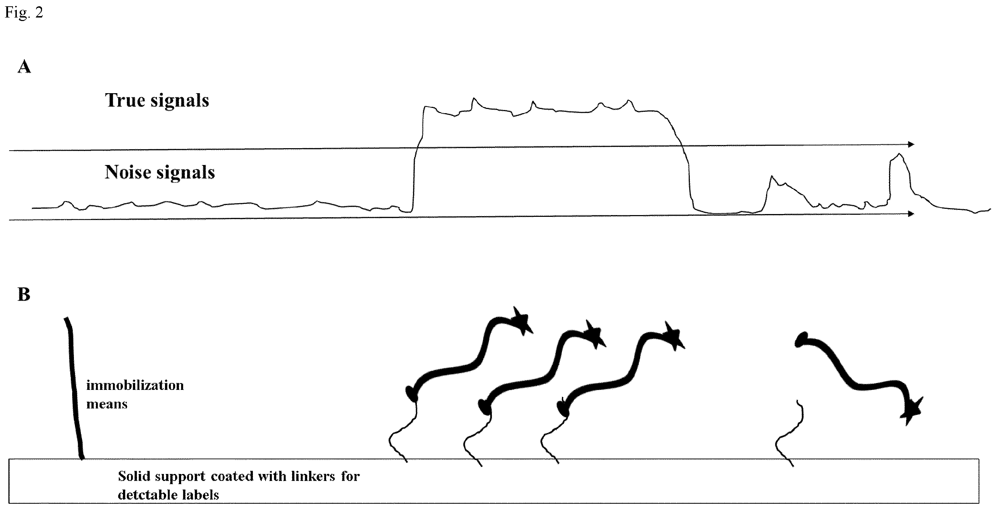

**Figure 2****:** (A) A schematic representation of measured signals from covalently bound detectable labels after removal of the immune complex. Only signals from covalently bound detectable labels in proximity to former immune complexes are detected as true signals having a certain intensity. Noise signals elicited by potentially unspecific bound or associated detectable label molecules remaining after washing are below the threshold. (B) Schematic representation of the underlying molecular setup on the solid support corresponding to the measured signals in (A).

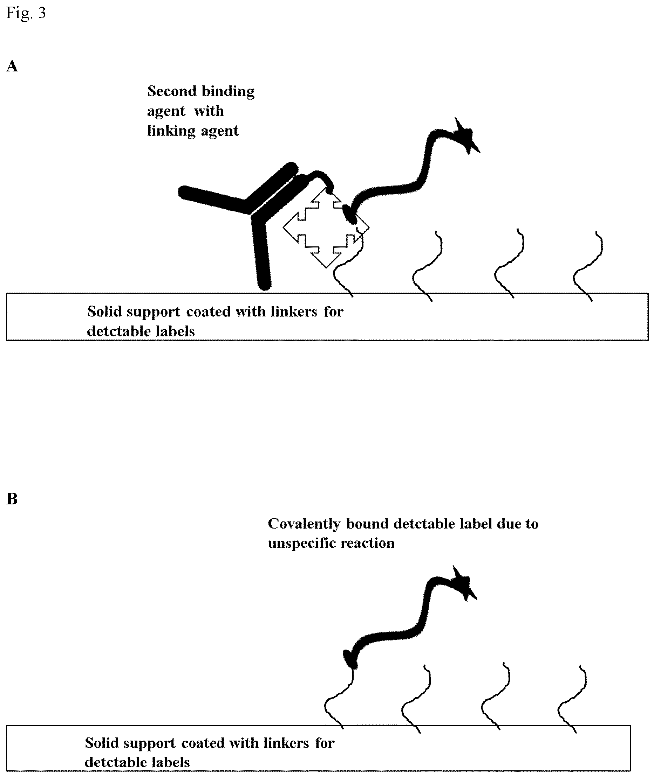

**Figure 3****:** (A) Undesired second binding agent in proximity to the solid support may also covalently bind some detectable labels. (B) Said covalently bound detectable labels may remain after harsh washing but are significantly less than those covalently bound to the solid support as a result of proper immune complex formation. The undesired covalently bound detectable labels result in a noise signal.

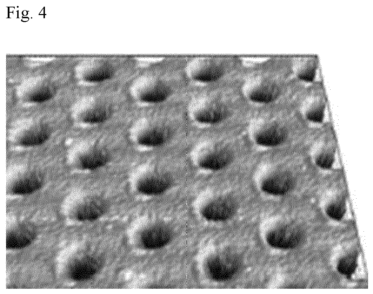

**Figure 4****:** Plasmonic surface with predefined subdivided areas for detecting single immune complex formations using a CCD camera read out.

## Claims

1. A method for determining an analyte suspected to be present in a sample comprising:
(a) contacting said sample with
(i) a first binding agent which is capable of specifically binding to the analyte and which is immobilized on a solid support; and
(ii) a second binding agent which is capable of specifically binding to the analyte and which is capable of reversibly binding at least one detectable label;
wherein said first and/or second binding agent is linked to an linking agent which is capable of covalently binding the at least one reversibly bound detectable label to the solid support when the first and second binding agents are in physical proximity,
for a time and under conditions which allow:
- specific binding of the analyte to the first and the second binding agent such that an complex of first binding agent, analyte and second binding agent is formed; and
- which allow for covalently binding the at least one detectable label to the solid support; and
(b) removing the complex of first binding agent, analyte and second binding agent from the solid support such that the solid support having covalently bound the at least one detectable label remains; and
(c) detecting the at least one covalently bound detectable label on the solid support whereby the analyte is determined.

2. The method of claim 1, wherein said first binding agent is selected from the group consisting of: an antibody or fragment thereof, an aptamer, a receptor molecule or fragment thereof, and a ligand molecule or fragment thereof.

3. The method of claim 1 or 2, wherein said second binding agent is selected from the group consisting of: an antibody or fragment thereof, an aptamer, a receptor molecule or fragment thereof, and a ligand molecule or fragment thereof.

4. The method of any one of claims 1 to 3, wherein said linking agent is a covalently cross-linking enzyme, preferably, a transglutaminase or sortase.

5. The method of any one of claims 1 to 3, wherein said linking agent comprises covalently cross-linking chemical moieties.

6. The method of any one of claims 1 to 5, wherein said detectable label is selected from the group consisting of: fluorescent labels, chemiluminescent label, radioactive labels, magnetic labels, and electrochemical labels.

7. The method of claim 6, wherein said detectable label comprises a linker which can be covalently linked by the linking agent to the solid support.

8. The method of any one of claims 1 to 7, wherein said solid support comprises on its surface a linker to which the label can be covalently bound by the linking agent.

9. The method of any one of claims 1 to 8, wherein said solid support is a bead, well or predetermined subdivided detection area, preferably, of a plasmonic resonator.

10. The method of claim 9, wherein each bead, well or predetermined subdivided detection area has immobilized a predefined amount of first binding agent.

11. The method of claim 10, wherein said determining an analyte comprises determining the amount of said analyte.

12. The method of claim 11, wherein said amount is determined by counting the beads, wells or predetermined subdivided detection areas which comprise at least one covalently bound detectable label.

13. A device for determining an analyte suspected to be present in a sample comprising
(a) a sensor element comprising:
(i) a first binding agent which is capable of specifically binding to the analyte and which is immobilized on a solid support; and
(ii) a second binding agent which is capable of specifically binding to the analyte and which is capable of reversibly binding at least one detectable label;
wherein said first and/or second binding agent is linked to an linking agent which is capable of covalently binding the at least one reversibly bound detectable label to the solid support when the first and second binding agents are in physical proximity; and
(b) a detector for detecting the at least one covalently bound detectable label on the solid support.

14. Use of the device of claim 13 for determining an analyte suspected to be present in a sample in said sample.

15. A kit for determining an analyte suspected to be present in a sample comprising:
(a) a first binding agent which is capable of specifically binding to the analyte and which is immobilized on a solid support;
(b) a second binding agent which is capable of specifically binding to the analyte and which is capable of reversibly binding at least one detectable label; wherein said first and/or second binding agent is linked to an linking agent which is capable of covalently binding the at least one reversibly bound detectable label to the solid support when the first and second binding agents are in physical proximity,
(c) a detectable label; and
(d) an agent for removing the complex of first binding agent, analyte and second binding agent from the solid support such that the solid support having covalently bound the at least one detectable label remains.

## Patentansprüche

1. Verfahren zum Bestimmen eines Analyten, der vermutlich in einer Probe vorliegt, umfassend:
(a) Inkontaktbringen der Probe mit
(i) einem ersten Bindemittel, das spezifisch an den Analyten binden kann und das auf einem festen Träger immobilisiert ist; und
(ii) einem zweiten Bindemittel, das spezifisch an den Analyten binden kann und das reversibel an mindestens eine nachweisbare Markierung binden kann;
wobei das erste und/oder das zweite Bindemittel mit einem Verknüpfungsmittel verknüpft sind/ist, das die mindestens eine reversibel gebundene nachweisbare Markierung kovalent an den festen Träger binden kann, wenn sich das erste und zweite Bindemittel in räumlicher Nähe befinden,
für eine Zeit und unter Bedingungen, die Folgendes ermöglichen:
- spezifisches Binden des Analyten an das erste und das zweite Bindemittel, sodass ein Komplex aus erstem Bindemittel, Analyt und zweitem Bindemittel gebildet wird; und
- die kovalentes Binden der mindestens einen nachweisbaren Markierung an den festen Träger ermöglichen; und
(b) Entfernen des Komplexes aus erstem Bindemittel, Analyt und zweitem Bindemittel von dem festen Träger, sodass der feste Träger, der die kovalent gebundene mindestens eine nachweisbare Markierung aufweist, zurückbleibt; und
(c) Nachweisen der mindestens einen kovalent gebundenen nachweisbaren Markierung auf dem festen Träger, wodurch der Analyt bestimmt wird.

2. Verfahren nach Anspruch 1, wobei das erste Bindemittel aus der Gruppe ausgewählt ist, die aus Folgendem besteht: einem Antikörper oder Fragment davon, einem Aptamer, einem Rezeptormolekül oder Fragment davon und einem Ligandenmolekül oder Fragment davon.

3. Verfahren nach Anspruch 1 oder 2, wobei das zweite Bindemittel aus der Gruppe ausgewählt ist, die aus Folgendem besteht: einem Antikörper oder Fragment davon, einem Aptamer, einem Rezeptormolekül oder Fragment davon und einem Ligandenmolekül oder Fragment davon.

4. Verfahren nach einem der Ansprüche 1 bis 3, wobei das Verknüpfungsmittel ein kovalent vernetzendes Enzym, vorzugsweise eine Transglutaminase oder Sortase, ist.

5. Verfahren nach einem der Ansprüche 1 bis 3, wobei das Verknüpfungsmittel kovalent vernetzende chemische Einheiten umfasst.

6. Verfahren nach einem der Ansprüche 1 bis 5, wobei die nachweisbare Markierung aus der Gruppe ausgewählt ist, die aus Folgendem besteht: fluoreszierenden Markierungen, chemilumineszierenden Markierungen, radioaktiven Markierungen, magnetischen Markierungen und elektrochemischen Markierungen.

7. Verfahren nach Anspruch 6, wobei die nachweisbare Markierung einen Linker umfasst, der mithilfe des Verknüpfungsmittels kovalent mit dem festen Träger verknüpft werden kann.

8. Verfahren nach einem der Ansprüche 1 bis 7, wobei der feste Träger auf seiner Oberfläche einen Linker umfasst, an den die Markierung mithilfe des Verknüpfungsmittels kovalent gebunden werden kann.

9. Verfahren nach einem der Ansprüche 1 bis 8, wobei der feste Träger ein Kügelchen, Well oder vorbestimmter unterteilter Nachweisbereich, vorzugsweise ein plasmonischer Resonator, ist.

10. Verfahren nach Anspruch 9, wobei an jedem Kügelchen, Well oder vorbestimmten unterteilten Nachweisbereich eine vordefinierte Menge an erstem Bindemittel immobilisiert ist.

11. Verfahren nach Anspruch 10, wobei das Bestimmen eines Analyten das Bestimmen der Menge des Analyten umfasst.

12. Verfahren nach Anspruch 11, wobei die Menge durch Zählen der Kügelchen, Wells oder vorbestimmten unterteilten Nachweisbereiche, die mindestens eine kovalent gebundene nachweisbare Markierung umfassen, bestimmt wird.

13. Vorrichtung zum Bestimmen eines Analyten, der vermutlich in einer Probe vorliegt, umfassend
(a) ein Sensorelement, umfassend:
(i) ein erstes Bindemittel, das spezifisch an den Analyten binden kann und das auf einem festen Träger immobilisiert ist; und
(ii) ein zweites Bindemittel, das spezifisch an den Analyten binden kann und das reversibel an mindestens eine nachweisbare Markierung binden kann;
wobei das erste und/oder das zweite Bindemittel mit einem Verknüpfungsmittel verknüpft sind/ist, das die mindestens eine reversibel gebundene nachweisbare Markierung kovalent an den festen Träger binden kann, wenn sich das erste und zweite Bindemittel in räumlicher Nähe befinden; und
(b) einen Detektor zum Nachweisen der mindestens einen kovalent gebundenen nachweisbaren Markierung auf dem festen Träger.

14. Verwendung der Vorrichtung nach Anspruch 13 zum Bestimmen eines Analyten, der vermutlich in einer Probe vorliegt, in der Probe.

15. Kit zum Bestimmen eines Analyten, der vermutlich in einer Probe vorliegt, umfassend:
(a) ein erstes Bindemittel, das spezifisch an den Analyten binden kann und das auf einem festen Träger immobilisiert ist;
(b) ein zweites Bindemittel, das spezifisch an den Analyten binden kann und das reversibel an mindestens eine nachweisbare Markierung binden kann;
wobei das erste und/oder das zweite Bindemittel mit einem Verknüpfungsmittel verknüpft sind/ist, das die mindestens eine reversibel gebundene nachweisbare Markierung kovalent an den festen Träger binden kann, wenn sich das erste und zweite Bindemittel in räumlicher Nähe befinden,
(c) eine nachweisbare Markierung; und
(d) ein Mittel zum Entfernen des Komplexes aus erstem Bindemittel, Analyt und zweitem Bindemittel von dem festen Träger, sodass der feste Träger, der die kovalent gebundene mindestens eine nachweisbare Markierung aufweist, zurückbleibt.

## Revendications

1. Procédé de détermination d'un analyte suspecté d'être présent dans un échantillon comprenant :
(a) la mise en contact dudit échantillon avec
(i) un premier agent de liaison qui est capable de se lier spécifiquement à l'analyte et qui est immobilisé sur un support solide ; et
(ii) un second agent de liaison qui est capable de se lier spécifiquement à l'analyte et qui est capable de se lier de manière réversible à au moins une étiquette détectable ;
dans lequel ledit premier et/ou second agent de liaison est lié à un agent de liaison qui est capable de lier de manière covalente l'au moins une étiquette détectable liée de manière réversible au support solide lorsque les premier et second agents de liaison sont à proximité physique,
pendant une durée et dans des conditions qui permettent :
- la liaison spécifique de l'analyte au premier et au second agent de liaison de telle sorte qu'un complexe du premier agent de liaison, de l'analyte et du second agent de liaison est formé ; et
- qui permettent une liaison covalente de l'au moins une étiquette détectable au support solide ; et
(b) le retrait du complexe du premier agent de liaison, de l'analyte et du second agent de liaison du support solide de telle sorte que le support solide auquel est liée de manière covalente l'au moins une étiquette détectable demeure ; et
(c) la détection de l'au moins une étiquette détectable liée de manière covalente sur le support solide moyennant quoi l'analyte est déterminé.

2. Procédé selon la revendication 1, dans lequel ledit premier agent de liaison est choisi dans le groupe constitué par : un anticorps ou un fragment de celui-ci, un aptamère, une molécule réceptrice ou un fragment de celle-ci et une molécule ligand ou un fragment de celle-ci.

3. Procédé selon la revendication 1 ou 2, dans lequel ledit second agent de liaison est choisi dans le groupe constitué par : un anticorps ou un fragment de celui-ci, un aptamère, une molécule réceptrice ou un fragment de celle-ci et une molécule ligand ou un fragment de celle-ci.

4. Procédé selon l'une quelconque des revendications 1 à 3, dans lequel ledit agent de liaison est une enzyme de réticulation covalente, de préférence, une transglutaminase ou une sortase.

5. Procédé selon l'une quelconque des revendications 1 à 3, dans lequel ledit agent de liaison comprend des fractions chimiques de réticulation covalente.

6. Procédé selon l'une quelconque des revendications 1 à 5, dans lequel ladite étiquette détectable est choisie dans le groupe constitué par : des étiquettes fluorescentes, une étiquette chimiluminescente, des étiquettes radioactives, des étiquettes magnétiques et des étiquettes électrochimiques.

7. Procédé selon la revendication 6, dans lequel ladite étiquette détectable comprend un lieur qui peut être lié de manière covalente par l'agent de liaison au support solide.

8. Procédé selon l'une quelconque des revendications 1 à 7, dans lequel ledit support solide comprend à sa surface un lieur auquel l'étiquette peut être liée de manière covalente par l'agent de liaison.

9. Procédé selon l'une quelconque des revendications 1 à 8, dans lequel ledit support solide est une bille, un puits ou une zone de détection subdivisée prédéterminée, de préférence, d'un résonateur plasmonique.

10. Procédé selon la revendication 9, dans laquelle chaque bille, puits ou zone de détection subdivisée prédéterminée a immobilisé une quantité prédéfinie du premier agent de liaison.

11. Procédé selon la revendication 10, dans lequel ladite détermination d'un analyte comprend la détermination de la quantité dudit analyte.

12. Procédé selon la revendication 11, dans lequel ladite quantité est déterminée en comptant les billes, les puits ou les zones de détection subdivisées prédéterminées qui comprennent au moins une étiquette détectable liée de manière covalente.

13. Dispositif de détermination d'un analyte suspecté d'être présent dans un échantillon comprenant
(a) un élément capteur comprenant :
(i) un premier agent de liaison qui est capable de se lier spécifiquement à l'analyte et qui est immobilisé sur un support solide ; et
(ii) un second agent de liaison qui est capable de se lier spécifiquement à l'analyte et qui est capable de se lier de manière réversible à au moins une étiquette détectable ;
dans lequel ledit premier et/ou second agent de liaison est lié à un agent de liaison qui est capable de lier de manière covalente l'au moins une étiquette détectable liée de manière réversible au support solide lorsque les premier et second agents de liaison sont à proximité physique ; et
(b) un détecteur pour détecter l'au moins une étiquette détectable liée de manière covalente sur le support solide.

14. Utilisation du dispositif selon la revendication 13 pour déterminer un analyte suspecté d'être présent dans un échantillon dans ledit échantillon.

15. Kit de détermination d'un analyte suspecté d'être présent dans un échantillon comprenant :
(a) un premier agent de liaison qui est capable de se lier spécifiquement à l'analyte et qui est immobilisé sur un support solide ;
(b) un second agent de liaison qui est capable de se lier spécifiquement à l'analyte et qui est capable de se lier de manière réversible à au moins une étiquette détectable ; dans lequel ledit premier et/ou second agent de liaison est lié à un agent de liaison qui est capable de lier de manière covalente l'au moins une étiquette détectable liée de manière réversible au support solide lorsque les premier et second agents de liaison sont à proximité physique,
(c) une étiquette détectable ; et
(d) un agent pour le retrait du complexe du premier agent de liaison, de l'analyte et du second agent de liaison du support solide de telle sorte que le support solide auquel est liée de manière covalente l'au moins une étiquette détectable demeure.
